# EUROPEAN PATENT APPLICATION

(11) **EP 1 518 929 A1**
(43) Date of publication of application: **30.03.2005**
(21) Application number: 03744510.3
(22) Date of filing: 06.03.2003
(51) Int. Cl.: C12N 15/12, C07K 14/47, C12Q 1/68, C12N 1/15, C12N 1/19, C12N 1/21, C12N 5/10, C07K 16/18

(54) **NOVEL GENES**

(30) Priority: 15.03.2002 JP 2002071592
(71) Applicant: KANEKA CORPORATION, Osaka-shi, Osaka 530-8288 (JP)
(72) Inventor: NIWA, Hideo, Akashi-shi, Hyogo 674-0084 (JP); YAMASHITA, Kenji, Takamatsu-shi, Kagawa 761-8003 (JP)
(74) Representative: HOFFMANN - EITLE
(86) International application number: PCT/JP2003/002620
(87) International publication number: WO 2003/078631

(57) **Abstract**

The object of the present invention is to detect and select insulin-producing cells capable of proliferating, and further to differentiate/proliferate insulin-producing cells and precursory thereof, or cells related thereto.

The present invention was made by finding three species of novel genes by detecting genes specifically expressed in pancreases of PHHI patients which are regarded as a model of spontaneous proliferation of pancreatic β cells, and searching for base sequence data bases.

According to the present invention, proliferating insulin-producing cells can be detected using these genes, gene products or gene sequences by, for example, Northern analysis or RT-PCR. Furthermore, these genes can be differentiated into insulin-producing cells by introducing these genes into appropriate cells in genetic engineering manner.

## Description

### TECHNICAL FIELD

The present invention relates to a technology of detecting proliferating insulin-producing cells from a tissue or cell population constituted of a plurality of cell species. The invention further relates to a technology of proliferating pancreatic β cells, which are insulin-producing cells, and cells precursory thereof or cells related to pancreatic β cells, for example nerve cells and the like.

### BACKGROUND ART

Pancreatic β cells are the only organ producing insulin, which is a peptide hormone capable of lowering the blood sugar level. When the insulin-producing ability of pancreatic β cells is impaired from some or other cause, it becomes impossible to maintain the blood sugar level within a normal range, resulting in the onset of diabetes. Transplanting cells capable of producing insulin into patients with diabetes whose insulin productivity has been impaired serves as a fundamental therapy for restoring the insulin productivity in them and maintaining their blood sugar levels within a normal range.

Conceivable as the source of supply of insulin-producing cells are dead body-derived pancreases, or cells capable of producing insulin differentiated from embryonic stem cells (ES cells) or mesenchymal stem cells, which are expected to be put into practical use in the near future. Embryonic stem cells are cells derived from the blastocyst inner cell mass and are capable of differentiating into almost all tissues or cells. Mesenchymal stem cells are pluripotent cells found in the bone marrow, blood, corium, periosteum, etc. It has been shown in recent years that these cells can be artificially caused to differentiate into such functional cells as insulin-producing cells, nerve cells and myocardial cells in vitro and in vivo. However, since the supply of such tissues or cell populations is limited, it is considered difficult to secure a number of cells sufficient to treat patients with diabetes. Therefore, proliferating insulin-producing cells or cells serving as the source of supply thereof is demanded.

A technique which possibly produce such effect comprises causing a cell differentiating/proliferating factor, such as HGF (hepatocyte growth factor), Reg protein or betacellulin, to act on such supply source cells (Otonkoski et al., Diabetes, vol. 43, pp. 947-953, 1994; Watanabe et al., Proc. Natl. Acad. Sci., vol. 91, pp. 3589-3592, 1994; Yamamoto et al., Diabetes, vol. 49, pp. 2021-2027, 2000). However, any factor capable of being applied for practical treatment purposes has not been found as yet.

Furthermore, if success is achieved in proliferating a cell population containing insulin-producing cells using such a cell differentiation/proliferation factor, it will be desired that truly effective cells alone, namely insulin-producing cells capable of proliferating alone, among the cell population be used for therapeutic purposes. Any method effective in selecting such cells has not been established as yet.

### SUMMARY OF THE INVENTION

It is an object of the present invention to provide a method of detecting the desired cells, namely insulin-producing cells capable of proliferating, from among a cell population containing a sufficient number of insulin-producing cells for therapeutic purposes using a cell differentiation/proliferation factor and, further, a method of selecting such cells and to provide a method of more efficiently differentiating/proliferating pancreatic β cells, which are insulin-producing cells, or cells related thereto, for example nerve cells and the like, for the treatment of diseases due to absolute insufficiency of a physiologically active substance to be produced in vivo, for example diabetes.

The present invention relates to a novel gene specifically expressed in the pancreas of PHHI patients, and the protein translated from that gene, and to a method of utilizing the same.

The protein of the invention is
(1) a protein
   which comprises the amino acid sequence shown under SEQ ID NO:1, 2 or 3 or
(2) a protein
   which has the amino acid sequence shown under SEQ ID NO:1, 2 or 3.
   The novel gene of the invention is
(1) a DNA
   which codes for the protein comprising the amino acid sequence shown under SEQ ID NO:1, 2 or 3,
(2) a DNA
   which comprises the base sequence shown under SEQ ID NO:4, 5 or 6, or
(3) a DNA which comprises the base sequence from the 174th to 904th base in SEQ ID NO:4,
   a DNA which comprises the base sequence from the 79th to 2115th base in SEQ ID NO:5, or
   a DNA which comprises the base sequence from the 28th to 384th base in SEQ ID NO:6, or
(4) a DNA comprising part of the base sequence shown under SEQ ID NO:4, 5 or 6
   which has at least the base sequence of DNA of the partial sequence region defined above under (3).

As the method of utilizing the novel gene and protein according to the invention, there may be mentioned
(1) a method of detecting proliferating insulin-producing cells
   which comprises using the novel DNA of the invention,
(2) a transformant cell
   which is obtainable by using a vector containing the novel DNA inserted therein,
(3) a method of differentiating embryonic stem cells or mesenchymal stem cells
   which comprises introducing the novel DNA into a primary cell or an established cell line comprising embryonic stem cells or mesenchymal stem cells, and
(4) a method of proliferating embryonic stem cells or mesenchymal stem cells
   which comprises introducing the novel DNA into a primary cell or an established cell line comprising embryonic stem cells or mesenchymal stem cells. In the method (3) or method (4), the embryonic stem cells or mesenchymal stem cells are preferably ones having an in vivo physiological function. Furthermore, in the method (3), the differentiated cells are preferably insulin-producing cells or nerve cells.

In another aspect, the invention provides
an antibody
which recognizes, as an antigen, the protein of the invention or the protein encoded by the novel gene.

In a further aspect, the invention provides
a method of diagnosing a disease
which utilizes said antibody. As the disease to be diagnosed by the diagnostic method of the invention, there may be mentioned diseases involving proliferative disease, pancreatic diseases, nervous system diseases, persistent hyperinsulinemic hypoglycemia of infancy, etc.

### DETAILED DESCRIPTION OF THE INVENTION

In the following, the present invention is described in detail.

In searching for a gene specifically expressed in proliferating insulin-producing cells or pancreatic β cells, the present inventors selected the pancreases of patients with persistent hyperinsulinemic hypoglycemia of infancy (PHHI) as tissues in which the expression of such a gene is highly possible. PHHI is a human hereditary disease also called nesidioblastosis and is known to involve a partial mutation of the potassium channel on pancreatic β cells. Therefore, the pancreatic β cells of patients with this disease are always stimulated to secrete insulin and the patients show severe symptoms of hypoglycemia (Science, 268, 426 (1995)). Furthermore, in PHHI patients, not only high blood insulin concentrations but also hyperplasia of islets of Langerhans, especially of pancreatic β cells, is observed, and such cells are clearly distinguishable from transformed cells such as cancer cells. Therefore, the pancreases of PHHI patients are regarded as a model of spontaneous proliferation of pancreatic β cells.

Therefore, the present inventors considered that if a gene specifically expressed in the pancreas of a PHHI patient could be identified, it would serve as a marker for detecting proliferating pancreatic β cells and, further, could code for a molecule causing the differentiation of precursor cells into pancreatic β cells or the proliferation of pancreatic β cells. Thus, they extracted RNA from the pancreases of PHHI patients and from the pancreases of normal subjects, synthesized cDNAs of the genes expressed in the respective tissues, performed gene subtraction using them, and successfully obtained novel genes specifically expressed in pancreases of PHHI patients.

Thus, the present invention relates to a novel gene specifically expressed in the pancreases of PHHI patients and the protein translated from the gene, and use thereof.

As the DNA of the invention, there may be mentioned
(1) a DNA
   which codes for the protein comprising the amino acid sequence shown under SEQ ID NO:1, 2 or 3,
(2) a DNA
   which comprises the base sequence shown under SEQ ID NO:4, 5 or 6, or
(3) a DNA which comprises the base sequence from the 174th to 904th base in SEQ ID NO:4, a DNA which comprises the base sequence from the 79th to 2115th base in SEQ ID NO:5, or a DNA which comprises the base sequence from the 28th to 384th base in SEQ ID NO:6, or
(4) a DNA comprising part of the base sequence shown under SEQ ID NO:4, 5 or 6
   which has at least the base sequence of DNA of the partial sequence region defined above under (3).

Here, the DNAs comprising the base sequence shown under SEQ ID NO:4, 5, or 6 as defined above under (2) are all novel genes found in the process of completion of the present invention. These novel genes could be obtained in the following manner.

RNA is extracted from each of PHHI patients' pancreases and normal subjects' pancreases by the acidic phenol method and, after purification of polyA(+) RNA, cDNAs originating from the respective tissues are synthesized using reverse transcriptase. Using these cDNAs as materials, gene subtraction is carried out by the method of Hubank and Schatz (Nucleic Acids Res., 22, 5640 (1993)), and the genes specifically expressed in the pancreases of PHHI patients are detected. This time, the base sequences of the specific genes were determined and searched for through base sequence data bases, and it was confirmed that at least three of the specific genes are novel genes, namely genes whose function is unknown.

The three genes confirmed to be novel genes as a result of database searching were respectively designated as NC1, NC2 and NC3. The base sequences thereof are shown under SEQ ID NO:4 (NC1), SEQ ID NO:5 (NC2) and SEQ ID NO:6 (NC3). The amino acid sequences of the proteins translated from those genes as deduced from the base sequences are shown under SEQ ID NO:1 (NC1), SEQ ID NO:2 (NC2) and SEQ ID NO:3 (NC3).

In the practice of the present invention, a DNA covering a part of the base sequence of the novel gene can also be utilized for the purposes mentioned later herein. The DNA covering a part of the base sequence, so referred to herein, is not particularly restricted if the intended purposes can be achieved. Specifically, however, there may be mentioned a DNA comprising the base sequence from the 174th to 904th base in SEQ ID NO:4, a DNA comprising the base sequence from the 79th to 2115th base in SEQ ID NO:5, or a DNA comprising the base sequence from the 28th to 384th base in SEQ ID NO:6 and, further, a DNA comprising a part of the base sequence shown under SEQ ID NO:4, 5 or 6 and containing said partial sequence region.

The method of preparing the DNA of the invention is not particularly restricted but the gene obtained by the above-mentioned method of obtaining the novel gene may be used as such or only a part thereof may be used. A DNA having said sequence may also be prepared by chemical synthesis.

As the protein of the invention, there may be mentioned (1) a protein which comprises the amino acid sequence shown under SEQ ID NO:1, 2 or 3 and (2) a protein which has the amino acid sequence shown under SEQ ID NO:1, 2 or 3. The method for obtaining these proteins is not particularly restricted but the proteins can be obtained, for example, in the manner of genetic engineering by using Escherichia coli, animal cells or the like as the host, transforming the same with a vector containing the above-mentioned DNA of the invention as inserted therein, and cultivating the resulting transformant. As for the method of insertion into the vector, the method of transformation, the method of cultivation, etc., the respective methods known in the art can be used.

When a DNA derived from a gene specifically expressed in the pancreases of PHHI patients and the protein translated from that DNA are used in accordance with the invention, proliferating insulin-producing cells/pancreatic β cells can be detected and selected from a tissue or cell population comprising various cell species by such methods as mentioned below.

RNA is extracted from a target tissue or cell population and subjected to northern analysis using, as a probe, the specific gene-derived DNA of the invention as labeled by an appropriate method, for example with the radioisotope ³²P and a DNA-modifying enzyme. If proliferating insulin-producing cells/pancreatic β cells are present in the target tissue or cell population, they are detected upon autoradiography. It is also possible to detect proliferating insulin-producing cells/pancreatic β cells by carrying out PCR using cDNA synthesized based on the RNA extracted from the target tissue or cell population as a template and the DNA of the invention as a primer.

Furthermore, it is possible to detect proliferating insulin-producing cells/pancreatic β cells from the target tissue or cell population by an immunological technique, for example by tissue immunostaining, using an antibody prepared in advance and recognizing the protein of the invention as an antigen. This antibody can also be used in diagnosing a disease in which the proliferation of insulin-producing cells/pancreatic β cells is involved. As such diseases, there may be mentioned, for example, diseases involving proliferative disease, pancreatic diseases, nervous diseases and the like. In particular, the above antibody is judiciously used in diagnosing persistent hyperinsulinemic hypoglycemia of infancy, among others.

Since the gene of the invention is specifically expressed in PHHI patients' pancreases, which is a model of spontaneous proliferation of pancreatic β cells, it is presumable that it is involved in the proliferation or differentiation of pancreatic β cells or cells related thereto. Therefore, when the DNA of the invention derived from said gene is introduced into insulin-producing cells/pancreatic β cells, into cells related thereto and precursor cells thereof, for example nerve cells or the like, or into cultured cells corresponding to such precursor cells, for example embryonic stem cells, mesenchymal stem cells or the like, and caused to be expressed, the proliferation of such cells can expectedly be promoted. Similarly, when the DNA of the invention is introduced into insulin-producing cells/pancreatic β cells, into cells related thereto and precursory thereof, for example nerve cells or the like, or into cultured cells corresponding to such precursor cells, for example embryonic stem cells, mesenchymal stem cells or the like, and caused to be expressed, the differentiation of such cells into insulin-producing cells or nerve cells can expectedly be induced. These embryonic stem cells or mesenchymal stem cells are preferably ones having an in vivo physiological function. The in vivo physiological function, so referred to herein, means a high level of in vivo or in vitro differentiating ability. As regards the method of introduction and expression, any of those known methods which are in general use can be employed.

### BRIEF DESCRIPTION OF THE DRAWINGS

Fig. 1 is a representation of the results of investigation, by northern analysis, of the expression of the NC1, NC2 and NC3 genes specific to the pancreases of patients with persistent hyperinsulinemic hypoglycemia of infancy.
Fig. 2 is a representation of the results of investigation, by northern analysis, of the changes in expression of the NC3 gene as resulting from cell differentiation.
Fig. 3 is a representation of the changes in morphology of PC12 cells after forced expression of the NC1 gene.

### BEST MODE FOR CARRYING OUT THE INVENTION

In the following, the present invention is described more specifically by means of examples. However, these examples are no limitative of the present invention without departing from the scope of the invention.

### (Example 1) Method for obtaining the novel genes NC1, NC2 and NC3

All RNA was extracted from a PHHI patient's pancrease and a normal subject's pancrease using RNAzolB (product of Biotecx Laboratories Inc.) to purify polyA(+) RNA using PolyATract messenger RNA isolation system (product of Promega). From this polyA(+) RNA, double strand cDNAs were synthesized using Riboclone cDNA Synthesis System (product of Promega). Each of the double strand cDNAs derived from the PHHI patient's pancrease and the normal subject's pancrease was completely cut by restriction enzyme DpnII (product of New England Biolabs Inc.). After stopping the reaction by phenol treatment, the cut cDNA fragments were recovered by ethanol precipitation. The recovered DNA fragments were suspended in sterilized distilled water, and 8 µg of R-Bgl-24 (oligo DNA having the base sequence of agcactctccagccctctcaccgca) and 4 µg of R-Bgl-12 (gatctgcggtga) were ligated to 1.2 µg of the cDNA fragment at 14°C for 16 hours using T4 DNA ligase (product of New England Biolabs Inc.) with a scale of 50 µl. The DNA concentration in the reaction product was adjusted to 6 µg/ml, and 1 µl of the product was fractionated and amplified by PCR reaction using R-Bgl-24 as a primer. The amplification product derived from the PHHI patient's pancrease is called as a tester, and the amplification product derived from the normal subject is called as a driver. The tester and driver were cut by DpnII to remove R-Bgl-24 and R-Bgl-12 oligo DNA, and to the tester alone, other oligo DNA J-Bgl-24 (accgacgtcgactatccatgaaca) and J-Bgl-12 (gatctgttcatg) were ligated. To 0.4 µg of the tester to which the oligo DNA has been ligated, centuple amount (40 µg) of the driver was added, and ethanol precipitation was carried out. Thereafter, the obtained product was suspended in 4 µl of EE x 3 buffer solution [30 mM N-(2-hydroxy-ethyl)piperazine-N'-3-propane sulfonic acid (product of Sigma); 3mM EDTA (pH 8.0)]. This DNA solution was denatured by heat treatment at 98°C for 5 minutes, kept at 67°C for 20 hours for annealing, and a TE buffer solution [10 mM Trizma Base (product of Sigma); 1mM EDTA (pH 8.0)] was added so as the total amount to be 400 µl. 10 µl of the mixture solution was fractionated, and kept at 72°C for 3 minutes to detach J-Bgl-12 from the cDNA. 5 units of Taq polymerase (product of New England Biolabs Inc.) were added thereto, and the resultant was further subjected to reaction for 5 minutes to modify the single strand part to a double strand. Using this reaction product as a mold, 10 cycles of PCR reaction was carried out at 95°C for 1 minute / 70°C for 3 minutes. The amplification product was subjected to phenol treatment and ethanol precipitation, and suspended in 400 µl of 0.2-fold TE buffer solution. 40 units of Mung Bean Nuclease (product of New England Biolabs Inc.) were added thereto, and the resultant was subjected to reaction at 30°C for 35 minutes. This reaction product is called as DP1. 2 µg of J-Bgl-24 was added to 10 µl of DP1 as a primer, and the resultant was subjected to 18 cycles of PCR reaction at 95°C for 1 minute / 70°C for 3 minutes. The obtained amplification product was cut by DpnII, 8 µg of N-Bgl-24 (aggcaactgtgctatcgagggaa) and 4 µg of N-Bgl-12 (gatcttccctcg) were ligated to 1.2 µg of the cDNA fragment, and the process for obtaining DP1 was followed to obtain DP2. DP2 was amplified by PCR reaction using N-Bgl-24 as a primer. The amplification product was cut by DpnII and ligated to J-Bgl-24 and J-Bgl-12 to obtain DP3 in the same manner. DP3 was amplified by PCR reaction (95°C for 1 minute / 70°C for 3 minutes, 22 cycles) using J-Bgl-24 as a primer. The obtained amplification product was cut by DpnII, and then the resultant was subcloned to BamHI site of pUC19. The base sequence of the obtained clone was determined, and the base sequence data bases such as Gen Bank were searched. As a result, three of those clones were not identical to various base sequences on the data bases. Furthermore, using these clones as probes, the cDNA library was screened to obtain full-length cDNA, and designated as NC1, NC2 and NC3.

### (Example 2) Detection of proliferating insulin cells/pancreatic β cells by Northern analysis

Using a part of the DNA in the gene sequences of NC1 (SEQ ID NO: 4), NC2 (SEQ ID NO: 5), and NC3 (SEQ ID NO: 6) obtained in Example 1, whether the objective tissues contain proliferating insulin-producing cells or not was investigated by Northern analysis.

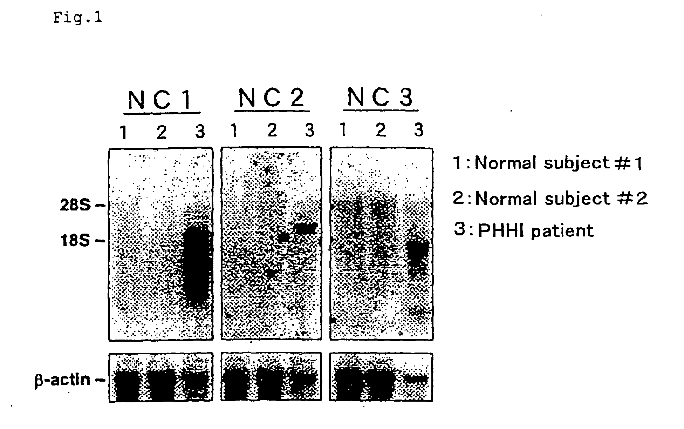

All RNA was extracted from normal subjects' pancreases (lane 1 and lane 2, two samples) and a PHHI patient's pancreas (lane 3, one sample) using TRIzol (product of GIBCO Lifetech Oriental), and then purified. After fractionating these by 1.0% agarose gel electrophoresis, the resultant was transferred onto a nylon membrane (Hybond-N, product of Amersham Pharmacia Biotech Inc.) by a capillary method. A DNA comprising the base sequence from the 174th to 904th base in SEQ ID NO:4, a DNA comprising the base sequence from the 79th to 2115th base in SEQ ID NO:5, and a DNA comprising the base sequence from the 28th to 384th base in SEQ ID NO:6 were amplified by a PCR method. After fractionating the resultant by agarose gel electrophoresis, the objective DNA fragment was cut from the gel and purified. These were radiolabeled using T4 polynucleotide kinase (product of TAKARA SHUZO CO., LTD) and [γ-32P]ATP (product of Amersham Pharmacia Biotech Inc.), and used as a probe for Northern analysis. The RNA-transferred nylon membrane and the radiolabeled probe were mixed and hybridized overnight at 65°C, the membrane was washed, and an RNA fraction reactive with the probe was detected by autoradiography (Fig. 1). When the DNAs of the partial sequences derived from NC1, NC2 and NC3 were used as probes, although a signal was detected from RNA derived from the PHHI patient, no signal was detected from RNA derived from the normal subject.

### (Example 3) Changes in expression of NC3 as resulting from cell differentiation

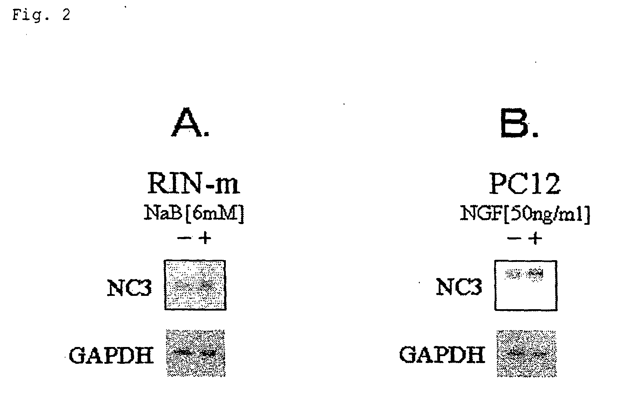

Although the insulin-producing ability of RIN-m cells established from rat insulinoma are low, it has been shown that the cells become to secrete insulin at high concentration by adding sodium butyrate to a cultivation system, and are differentiated into insulin-producing cells (Bartholomeusz et al., Endocrinology vol. 24, 2680-2685 pages, 1989). After RIN-m cells were cultivated in the presence of 6 mM sodium butyrate for 16 hours, all RNA was extracted, and Northern analysis was carried out in the same manner as described in Example 2 using a DNA comprising the base sequences from the 28th to 384th base in SEQ ID:6 as a probe. As a result, as shown in A of Fig. 2, the expression of NC3 gene was promoted in RIN-m cells which have been cultivated by adding sodium butyrate.

Furthermore, PC-12 cells derived from adrenal pheochromocytoma are known to elongate the neurite by adding nerve growth factor (NGF), and to differentiate into nerve cell like (Saltiei et al., Bioessay vol.16, 405-411 pages, 1994). All RNA was extracted from PC-12 cells which have been cultivated for 16 hours in the cultivation system added with NGF to have a concentration of 50 ng/ml. A DNA comprising the base sequence from the 28th to 384th base in SEQ ID NO:6 was used as a probe, and Northern analysis was carried out in the same manner. As shown in B of Fig. 2, the expression of NC3 gene was promoted in PC-12 cells which have been cultivated with an addition of NGF.

### (Example 4) Changes in morphology of PC12 cells after forced expression of NC1 gene

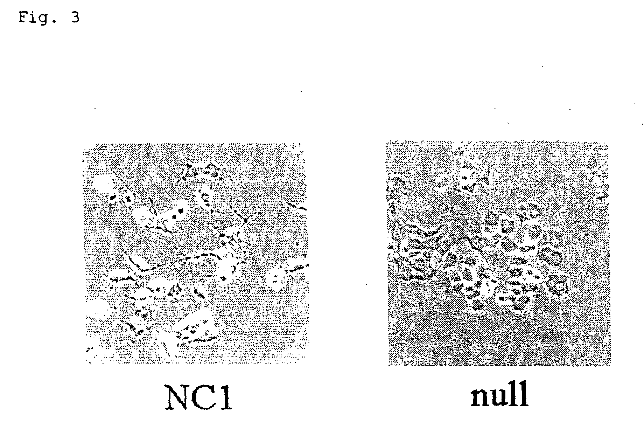

Among the base sequences shown under SEQ ID NO:4, a DNA of the base sequence from the 174th to 904th base, which is a region coding for a protein comprising the amino acid sequence of the SEQ ID NO:1, was inserted into a predetermined site of pCIneo, which is a gene expression vector for animal cells, and thereby an NC1 gene expression vector was constructed. This was introduced into PC-12 cells by a liposome method to carry out forced expression. The cells were kept in the cultivation system to which geneticin has been added at a concentration of 500 µg/ml, and the cells having the expression vector were selected. As shown in Fig. 3, cells elongating neurites were observed in NC1 gene expression-forced PC-12 cells, and it was suggested that PC-12 cells could be differentiated into nerve cell like by forced expression of NC1 gene.

### INDUSTRIAL APPLICABILITY

The novel genes found in the present invention are not only specifically expressed in PHHI patients' pancreases, but also changing its expression over cell differentiation and/or proliferation, and capable of inducing differentiation into cells having a function by forcibly expressing into undifferentiated cells in genetic engineering manner. Accordingly, it becomes possible to easily detect insulin-producing cells capable of proliferating by using these genes, a part of the DNA and a protein translated therefrom, to select them, and further to differentiate and proliferate insulin-producing cells. Furthermore, it becomes possible to develop a novel diagnostic method of various diseases resulting from abnormal differentiation and proliferation of pancreatic β cells, which are insulin-producing cells, and cells related to pancreatic β cells (for example nerve cells).

SEQUENCE LISTING

<110> KANEKA CORPORATION

<120> NOVEL GENES

<130> T725.TEMNC-1

<150> JP P2002-071592
<151> 2002-03-15

<160> 6

<210> 1
<211> 247
<212> PRT
<213> Homo sapiens

<220>
<223> NC1

<400> 1
Met Gln Val Val Lys Glu Gln Val Met Arg Ala Leu Thr Thr Lys Pro
1               5                   10                  15
Ser Ser Leu Asp Gln Phe Lys Ser Lys Leu Gln Asn Leu Ser Tyr Thr
            20                  25                  30
Glu Ile Leu Lys Ile Arg Gln Ser Glu Arg Met Asn Gln Glu Asp Phe
        35                  40                  45

Gln Ser Arg Pro Ile Leu Glu Leu Lys Glu Lys Ile Gln Pro Glu Ile
    50                  55                  60
Leu Glu Leu Ile Lys Glu Glu Arg Leu Asn Arg Leu Val Glu Gly Thr
65                  70                  75                  80
Cys Phe Arg Lys Leu Asn Ala Arg Arg Arg Gln Asu Lys Phe Trp Tyr
85 90 95
Cys Arg Lys Ser Pro Asn His Lys Val Leu His Tyr Gly Asu Leu Glu
100 105 110
Glu Ser Pro Gln Gly Glu Val Pro His Asu Ser Leu Gln Asu Lys Leu
115 120 125
Pro Val Ala Asu Ile Lys Ala Val Val Thr Gly Lys Asu Cys Pro His
130 135 140
Met Lys Glu Lys Gly Ala Leu Lys Gln Asn Lys Glu Val Leu Glu Leu
145 150 155 160
Ala Phe Ser Ile Leu Tyr Asu Ser Asn Cys Gln Leu Asn Phe Ile Ala
165 170 175
Pro Asu Lys His Glu Tyr Cys Ile Trp Thr Asu Gly Leu Asn Ala Leu
180 185 190
Leu Gly Lys Asu Met Met Ser Asu Leu Thr Arg Asn Asu Leu Asu Thr
195 200 205
Leu Leu Ser Met Glu Ile Lys Leu Arg Leu Leu Asu Leu Glu Asn Ile
210 215 220
Gln Ile Pro Asu Ala Pro Pro Pro Ile Pro Lys Glu Pro Ser Asn Tyr
225 230 235 240
Asp Phe Val Tyr Asu Cys Asn
245

<210> 2

<211> 679

<212> PRT

<213> Homo sapiens

<220>

<223> NC2

<400> 2

Met Asp Arg Val Thr Arg Tyr Pro Ile Leu Gly Ile Pro Gln Ala His
1               5                   10                  15

Arg Gly Thr Gly Leu Val Leu Asp Gly Asp Thr Ser Tyr Thr Tyr His
            20                  25                  30

Leu Val Cys Met Gly Pro Glu Ala Ser Gly Trp Gly Gln Asp Glu Pro
        35                  40                  45

Gln Thr Trp Pro Thr Asp His Arg Ala Gln Gln Gly Val Gln Arg Gln
    50                  55                  60

Gly Val Ser Tyr Ser Val His Ala Tyr Thr Gly Gln Pro Ser Pro Arg
65                  70                  75                  80

Gly Leu His Ser Glu Asn Arg Glu Asp Glu Gly Trp Gln Val Tyr Arg
                85                  90                  95

Leu Gly Ala Arg Asp Ala His Gln Gly Arg Pro Thr Trp Ala Leu Arg
            100                 105                 110

Pro Glu Asp Gly Glu Asp Lys Glu Met Lys Thr Tyr Arg Leu Asp Ala
        115                 120                 125

Gly Asp Ala Asp Pro Arg Arg Leu Cys Asp Leu Glu Arg Glu Arg Trp
    130                 135                 140

Ala Val Ile Gln Gly Gln Ala Val Arg Lys Ser Ser Thr Val Ala Thr
145                 150                 155                 160

Leu Gln Gly Thr Pro Asp His Gly Asp Pro Arg Thr Pro Gly Pro Pro
                165                 170                 175
Arg Ser Thr Pro Leu Asp Asp Asn Val Val Asp Arg Glu Gln Ile Asp
            180                 185                 190
Phe Leu Ala Ala Arg Gln Gln Phe Leu Ser Leu Glu Gln Ala Asn Lys
        195                 200                 205
Gly Ala Pro His Ser Ser Pro Ala Arg Gly Thr Pro Ala Gly Thr Thr
    210                 215                 220
Pro Gly Ala Ser Gln Ala Pro Lys Ala Phe Asn Lys Pro His Leu Ala
225                 230                 235                 240
Asn Gly His Val Val Pro Ile Lys Pro Gln Val Lys Gly Val Val Arg
                245                 250                 255
Glu Glu Asn Lys Val Arg Ala Val Pro Thr Trp Ala Ser Val Gln Val
            260                 265                 270
Val Asp Asp Pro Gly Ser Leu Ala Ser Val Glu Ser Pro Gly Thr Pro
        275                 280                 285
Lys Glu Thr Pro Ile Glu Arg Glu Ile Arg Leu Ala Gln Glu Arg Glu
    290                 295                 300
Ala Asp Leu Arg Asp Gln Arg Gly Leu Arg Gln Ala Thr Asp His Gln
305                 310                 315                 320
Glu Leu Val Glu Ile Pro Thr Arg Pro Leu Leu Thr Lys Leu Ser Leu
                325                 330                 335
Ile Thr Ala Pro Arg Arg Glu Arg Gly Arg Pro Ser Lys Tyr Val Gln
            340                 345                 350
Arg Asp Ile Val Gln Glu Thr Gln Arg Glu Glu Asp His Arg Arg Glu
        355                 360                 365
Gly Lys His Val Gly Arg Ala Ser Thr Pro Asp Trp Val Ser Glu Gly
    370                 375                 380

Pro Gln Pro Gly Leu Arg Arg Ala Leu Ser Ser Asp Ser Ile Leu Ser
385                 390                 395                 400
Pro Ala Pro Asp Ala Arg Ala Ala Asp Pro Ala Pro Glu Val Arg Lys
                405                 410                 415
Val Asn Arg Ile Pro Pro Asp Ala Tyr Gln Pro Tyr Leu Ser Pro Gly
            420                 425                 430
Thr Pro Gln Leu Glu Phe Ser Ala Phe Gly Ala Phe Gly Lys Pro Ser
        435                 440                 445
Ser Leu Ser Thr Ala Glu Ala Lys Ala Ala Thr Ser Pro Lys Ala Thr
    450                 455                 460
Met Ser Pro Arg His Leu Ser Glu Ser Ser Gly Lys Pro Leu Ser Thr
465                 470                 475                 480
Lys Gln Glu Ala Ser Lys Pro Pro Arg Gly Cys Pro Gln Ala Asn Arg
                485                 490                 495
Gly Val Val Arg Trp Glu Tyr Phe Arg Leu Arg Pro Leu Arg Phe Arg
            500                 505                 510
Ala Pro Asp Glu Pro Gln Gln Ala Gln Val Pro His Val Trp Gly Trp
        515                 520                 525
Glu Val Ala Gly Ala Pro Ala Leu Arg Leu Gln Lys Ser Gln Ser Ser
    530                 535                 540
Asp Leu Leu Glu Arg Glu Arg Glu Ser Val Leu Arg Arg Glu Gln Glu
545                 550                 555                 560
Val Ala Glu Glu Arg Arg Asn Ala Leu Phe Pro Glu Val Phe Ser Pro
                565                 570                 575
Thr Pro Asp Glu Asn Ser Asp Gln Asn Ser Arg Ser Ser Ser Gln Ala
            580                 585                 590
Ser Gly Ile Thr Gly Ser Tyr Ser Val Ser Glu Ser Pro Phe Phe Ser
        595                 600                 605

Pro Ile His Leu His Ser Asn Val Ala Trp Thr Val Glu Asp Pro Val
    610                 615                 620
Asp Ser Ala Pro Pro Gly Gln Arg Lys Lys Asp Gln Trp Tyr Ala Gly
625                 630                 635                 640
Ile Asn Pro Ser Asp Gly Ile Asn Ser Glu Val Leu Glu Ala Ile Arg
                645                 650                 655
Val Thr Arg His Lys Asn Ala Met Ala Glu Arg Trp Glu Ser Arg Ile
            660                 665                 670
Tyr Ala Ser Glu Glu Asp Asp
        675

<210> 3
<223> 119
<212> PRT
<213> Homo sapiens

<220>
<223> NC3

<400> 3
Met Ala Asp Gly Leu Phe Arg Arg Arg Pro Trp Gly Leu Glu Gln Ile
1               5                   10                  15
Arg Pro Asp Pro Glu Ser Glu Gly Leu Phe Asp Lys Pro Pro Pro Glu
            20                  25                  30
Asp Pro Pro Ala Ala Arg Gly Pro Arg Ser Ala Ser Ala Ala Gly Lys
        35                  40                  45
Lys Ala Gly Arg Arg Ala Gly Gly Arg Ala Gln Gly Gly Arg Ala Gly

    50                  55                  60

Gln Pro Pro Lys Ala Ala Ser Arg Pro Pro Pro Lys Lys Glu Ala Pro

65                  70                  75                  80

Pro Leu Asp Glu Gly Cys Tyr Leu Asp His Phe Pro His Leu Ser Ile

                85                  90                  95

Phe Ile Tyr Ala Ala Ile Ala Phe Ser Ile Thr Ser Cys Ile Phe Thr

            100                 105                 110

Tyr Ile His Leu Gln Leu Ala

        115

<210> 4

<211> 2109

<212> DNA

<213> Homo sapiens

<220>

<221> CDS

<222> (174)...(904)

<223> NC1

<400> 4

atgttcacat ggctcaactg gaaacctgtt tcatgaacaa gcttactcag gaaccatctg 60

gtggtattcc agcacattgt tcttcagggg gacgactcta agtcgctttg tggtggcagc 120

agcttagaat cagtatttgt ggttgggaaa gatggactta cgggagcttg gtaatgcagg 180

tggtgaagga gcaggttatg agagcactta caaccaagcc tagctccctg gaccagttca 240

agagcaaact gcagaacctg agctacactg agatcctgaa aatccgccag tccgagagga 300

tgaaccagga agatttccag tcccgcccga ttttggaact aaaggagaag attcagccag 360

aaatcttaga gctgatcaaa cagcaacgcc tgaaccgcct tgtggaaggg acctgcttta 420
ggaaactcaa tgcccggcgg aggcaagaca agttttggta ttgtcggctt tcgccaaatc 480
acaaagtcct gcattacgga gacttagaag agagtcctca gggagaagtg ccccacgatt 540
ccttgcagga caaactgccg gtggcagata tcaaagccgt ggtgacggga aaggactgcc 600
ctcatatgaa agagaaaggt gcccttaaac aaaacaagga ggtgcttgaa ctcgctttct 660
ccatcttgta tgactcaaac tgccaactga acttcatcgc tcctgacaag catgagtact 720
gtatctggac agatggactg aatgcgctac tcggaaggga catgatgagc gacctgacgc 780
ggaatgacct ggacaccctg ctcagcatgg aaatcaagct ccgcctcctg gacctggaaa 840
acatccagat ccctgacgca cctccgccga ttcccaagga gcccagcaac tatgacttcg 900
tctatgactg taactgaagt ggccgggccc agacatgccc cttccaaaac tggaacacct 960
agctaacagg agagaggaat gaaaacacac ccacgccttg gaaccgtcct ttggtaaagg 1020
gaagctgtgg gtccacattc ccttcagcat cacctctagc cctggcaact ttcagcccct 1080
agctggcatc ttgctcaccg ccctgattct gttcctcggc tccactgctt caggtcactt 1140
cccatggctg cagtccactg gtgggacaag agcaaagccc actgccagta agaaggccaa 1200
agggcccttc catcctagcc ctctgcaggc atgcccttcc ttcccttggg caggaaagcc 1260
agcagcccca gactgcccaa aaacttgccc accagaccaa gggcagtgcc ccaaggcccc 1320
tgtctggagg aaatggccta gctatttgat gagaagacca aaccccacat cctcctttcc 1380
cctctctcta gaatcatctc gcaccaccag ttacacttga attaagatct gcgctcaaat 1440
ctcctcccac ctctctccct gcttttgcct tgctctgttc ctctttggtc ccaagagcag 1500
cagccgcagc ctcctcgtga tcctccctag cataaatttc ccaaacagtc cacaggtccc 1560
atgcccactt tgcgtctgca ctgtgatcgt gacaaatctt ccctcctcac cagctagtct 1620
ggggtttcct ctccctgccc caggccagaa ctgccttctt catttccacc cacgctccca 1680
gcctcttagc tgaaagcaca aatggtgaaa tcagtagtct cgctccatct ctaatagact 1740
aaacctaaat gcctctagga cggactgttg ctatccaagc gtttggtgtt accttctcct 1800
gggaggtcct gctgcaactc aagttccaca ggatggtcaa gctgtcagac atccaagttt 1860
acatcattgt aattattact ggtatttaca atttgcaaga gttttgggtt agtttttttt 1920
tttttttttt tgctttgttt ttgtacaaaa gagtctaaca tttttgccaa aacagatata 1980
tatttaatga aaagaagaga tacataaatg tgtgaatttc cagttttttt tttaattatt 2040

ttaatcccaa acatcttcct gaaaataaca ttcccttaaa catgctgtgg aataaaatgg 2100
attgtgatg 2109

<210> 5
<211> 2846
<212> DNA
<213> Homo sapiens

<220>
<221> CDS
<222> (79)...(2115)
<223> NC2

<400> 5
tttccttctc ctccctcagt aagcccagag gtctccaccc cacgggagga aggctgaggc 60
caagaccccg gaagagatat ggaccgcgtg accagatacc ccatcctggg catccctcag 120
gcacaccgtg gcaccggcct ggtgctggat ggagacacca gctacacata ccatctggtg 180
tgcatgggcc ccgaggccag cggctggggc caggatgagc cgcagacatg gcccactgac 240
cacagggccc agcagggcgt gcagaggcag ggggtgtcct acagcgtgca tgcctacact 300
ggccagccgt ccccacgggg gctccactcg gagaacaggg aggatgaggg ttggcaggtt 360
taccgcctgg gcgccaggga tgcccaccag ggacgtccaa catgggcact ccgcccagag 420
gacggggagg acaaggagat gaagacctac cgcctggatg ctggggacgc tgaccccagg 480
aggctgtgtg acctggagcg ggagcgctgg gccgtcatcc agggccaggc agtcaggaag 540
agcagcaccg tggccacgct ccagggcact cctgaccacg gagaccccag gacccccggc 600
ccacctcggt ccacgcccct ggaggagaac gtggttgaca gggagcagat tgacttcctg 660
gcagcgagac agcagttcct gagtctggag caggcgaaca agggggcccc tcatagctcc 720
ccggccaggg ggacccctgc aggcacaacc ccaggggcca gccaggcccc caaggccttc 780
aacaagcccc acctggccaa cgggcacgtg gttcccatca agccccaggt gaagggggtg 840

gtcagggaag agaacaaggt gcgtgctgtg cccacctggg ccagtgtcca agttgtggat 900
gaccctggct ccttggcctc agtggagtcc ccggggaccc ccaaggagac gcccatcgag 960
cgggagatcc gtctggctca ggagcgtgag gcagacctgc gagagcagag ggggcttcgg 1020
caggcaaccg accaccagga gctggtggaa atccccacca ggccgctgct gaccaagctg 1080
agcctgatca cagccccacg gcgggagaga gggcgcccgt ccctctacgt gcagcgggac 1140
atagtacagg agacacagcg tgaggaagac caccggcggg agggcctgca cgtgggccgg 1200
gcgtccacac ccgactgggt ctcggagggt ccccagcccg gactccggag agccctcagc 1260
tcagattcca tcctcagccc ggccccagat gcccgtgcgg ccgacccagc tccagaagtg 1320
aggaaggtga accgcatccc acctgatgcc taccagccgt acctgagccc cgggaccccc 1380
cagctagaat tctcagcctt cggagcattc ggcaagccca gcagtctctc cacagcggag 1440
gccaaggctg cgacttcacc aaaggccacg atgtccccga ggcatctctc agaatcctct 1500
ggaaaacccc tgagcacaaa gcaagaggca tcgaagcccc ctcggggatg cccgcaagcc 1560
aacaggggtg tcgtgcggtg ggagtacttc cgcctgcgtc ctctgcggtt cagggcccca 1620
gacgagcccc agcaggccca agtcccccat gtctggggct gggaggtggc tggggcccct 1680
gcactgaggc tgcagaagtc ccagtcatct gatctgctgg aaagggagag ggagagtgtc 1740
ctgcgccggg agcaagaggt ggcagaggag cggagaaatg ctctcttccc agaggtcttc 1800
tccccaacgc cagatgagaa ctctgaccag aactccagga gctcctccca ggcatccggc 1860
atcacgggca gttactcggt gtctgagtct cccttcttca gccccatcca cctacactca 1920
aacgtggcgt ggacagtgga agatccagtg gacagtgctc ctcccgggca gagaaagaag 1980
gagcaatggt acgctggcat caacccctcg gacggtatca actcagaggt cctggaagcc 2040
atacgggtga cccgtcacaa gaacgccatg gcagagcgct gggaatcccg catctacgcc 2100
agtgaggagg atgactgagc ctcgggatgg ggcgcccacc ccctgccctg ccctgaccct 2160
cgtgggaact gccaagacca tcgccaagcc cccaccctag gaaatgggtc ctaggtccag 2220
gatccaagaa ccacagctca tctgccaaca atcccaccat gggcacattt gggactgttg 2280
ggtttttcgt ttccgtttct atcttccttt agaaatgttt ctgcctttgg ggtctaaagc 2340
ttttggggat gaaatgggac ccctgctgat tctttctgct tctaagactt tgccaaatgc 2400
cctgggtcta agaaagaaag agacccgctc ctccactttc aggtgtaatt tgcttccgct 2460
agtctgaggg cagagggacc ggtcaaagag ggtggcacag atcgcagcac cttgaggggc 2520

tgcgggtctg agggaggaga cactcagctc ctccctctga gaagtcccaa gctgagaggg 2580
gagacctgcc cctttccaac cctgggaaac catccagtct gagggaggag gccaaactcc 2640
cagtgctggg ggtccctgtg cagccctcaa acccttcacc ttggtgcacc cagccacacc 2700
tggtggacac aaagctctca catcgatagg atcccatgag gatggtcccc ttcacctggg 2760
agaaaagtga cccagtttag gagctggagg ggggtctttg tcccccaccc ccaaactgcc 2820
ctgaaataaa cctggagtga gctgcc 2846

<210> 6
<211> 1556
<212> DNA
<213> Homo sapiens

<220>
<221> CDS
<222> (28)...(384)
<223> NC3

<400> 6
ctgacccacc tacccgcgat cctgcccatg gctgacgggc tctttcggcg cagaccctgg 60
ggtctccagc agattcgccc ggaccccgag tccgaaggcc tgtttgacaa gcctcccccg 120
gaagaccctc ccgctgcccg cgggcccagg tcggcgtcgg ccgcgggcaa gaaggctggt 180
cggcgcgcgg gcgggagggc gcaggggggc cgcgccgggc agcccccgaa ggccgcatcg 240
cgccccccgc ccaagaagga ggcgcctcca ctggacgagg gctgctatct cgaccatttt 300
ccgcacctct ccatcttcat ctacgcagcc atcgccttct ccatcacctc ctgcatcttt 360
acctatatcc atttacagct tgcctgagtg gccagcgcgg gacggggtgg gcgcaggacc 420
gagcggggag ggaaagggaa aacggggctc ggcattttgt gttttagaac agcgctgcac 480
ccccttcatg tagctttcga tgcttgtttc tttccgtctt tgttgtcact atctttgtct 540
atcagtacga aagtacaaag tagctgccgg caatgaaata ggggtgctgt ttgcacctgc 600

aggttagggg tggaggcgtt tagaattttg gggtgtgatt gagccccgtt tataattaga 660
atgcccctgg acccctacca ctctgtgacg tgggggcacg cgcagggatc ccatcatttt 720
gtgtttgggg agctcagagt gcgcccaatc ttggaatctt taagggatga gccagaccca 780
gacccgcggc cttctagaga gggtccggca gggagggtcg gcgccctggc ccggggtggg 840
ccggagccct gtgatgctgc atcgccccca ggaggagcca gctgtgcccc agagttggcg 900
cggccgagag aggacaagag cgcgcagcag gcgaagctgg agggcgggac tcggtaagtg 960
gcgttcgtcg gggtgtcgtg ctgcgccccc aggggctccg gctgaccacg actgtgtgtt 1020
tttcctgcct tagactttgt tgtcgctgcc cggaggagtc gagactggta cccggaggag 1080
ctgtctcacc aggagaccac gtcctggaag tgtccgggac tcgcgggcgg tgtggctgca 1140
gaccccgccg gcacgcaggc ccagagctgg cgcactcctg aggatgagac tctgggggcc 1200
ctagccgggg tccacgggag ggctgtcctt ggggactcta ggatggcttc gttctggccc 1260
ggctcacttc tggagctgtg agacccaaga caaaaggggc tgagggattt ctcattgaca 1320
agggttcgtg cgggaaaacc acatgatccc tgggatttgt catcttaaga ctcaaaaggc 1380
ttaataccag gaaccacctt ggcaagatat tttacccacc ggccatctct gtttactcat 1440
gaatgttaaa tgttaaaacg cagcgctcta accctgcata ttatttactt gcaaatgtct 1500
gtaatctgta attgtgatgc ctctgatgga ataaattatc tttttcagtc tcctct 1556

## Claims

1. A protein
which consists of the amino acid sequence shown under SEQ ID NO:1, 2 or 3.

2. A protein
which comprises the amino acid sequence shown under SEQ ID NO:1, 2 or 3.

3. A DNA
which codes for the protein comprising the amino acid sequence shown under SEQ ID NO:1, 2 or 3.

4. A DNA
which comprises the base sequence shown under SEQ ID NO:4, 5 or 6.

5. A DNA which comprises the base sequence from the 174th to 904th base in SEQ ID NO:4,
a DNA which comprises the base sequence from the 79th to 2115th base in SEQ ID NO:5, or
a DNA which comprises the base sequence from the 28th to 384th base in SEQ ID NO:6.

6. A DNA comprising part of the base sequence shown under SEQ ID NO:4, 5 or 6
which has at least the base sequence of DNA according to Claim 5.

7. A method of detection of a proliferating insulin-producing cell
which comprises using the DNA according to any one of Claims 3 to 6.

8. A transformant cell
which is obtainable by using a vector containing the DNA according to any one of Claims 3 to 6 inserted therein.

9. A method of differentiation of an embryonic stem cell or a mesenchymal stem cell
which comprises introducing the DNA according to any one of Claims 3 to 6 into primary cells or established cell lines having an embryonic stem cell or a mesenchymal stem cell.

10. A method of proliferation of an embryonic stem
cell or a mesenchymal stem cell
which comprises introducing the DNA according to any one of Claims 3 to 6 into a primary cell or an established cell line having an embryonic stem cell or a mesenchymal stem cell.

11. The method according to Claim 9 or 10,
wherein the embryonic stem cell or mesenchymal stem cell has an in vivo physiological function.

12. The method according to Claim 9,
wherein the differentiated cell is an insulin-producing cell.

13. The method according to Claim 9,
wherein the differentiated cell is a nerve cell.

14. An antibody
which recognizes, as an antigen, any one of the following proteins (a) to (c):
(a) the protein according to Claim 1;
(b) the protein according to Claim 2; and
(c) the protein encoded by the DNA according to any one of Claims 3 to 6.

15. A method of diagnosing a disease
which utilizes the antibody according to Claim 14.

16. The method of diagnosing a disease according to Claim 15,
wherein the objective disease is a proliferative disease.

17. The method of diagnosing a disease according to Claim 15,
wherein the objective disease is a pancreatic disease.

18. The method of diagnosing a disease according to Claim 15,
wherein the objective disease is a nervous system disease.

19. The method of diagnosing a disease according to Claim 15,
wherein the objective disease is persistent hyperinsulinemic hypoglycemia of infancy.
